# EUROPEAN PATENT SPECIFICATION

(11) **EP 3 006 004 B1**
(45) Date of publication and mention of the grant of the patent: **22.07.2020**
(21) Application number: 13886119.0
(22) Date of filing: 28.05.2013
(51) Int. Cl.: A61G 5/14, A61B 5/024, A61H 3/04, A61H 3/00, B25J 11/00, B25J 9/16

(54) **ASSISTANCE ROBOT**
HILFSROBOTER
ROBOT D'ASSISTANCE

(43) Date of publication of application: 13.04.2016
(73) Proprietor: FUJI Corporation, Chiryu Aichi (JP)
(72) Inventor: NOMURA, Hideaki, Chiryu Aichi (JP); ISOZUMI, Joji, Chiryu Aichi (JP); NAKANE, Nobuyuki, Chiryu Aichi (JP)
(74) Representative: Grünecker Patent- und Rechtsanwälte PartG mbB
(86) International application number: PCT/JP2013/064805
(87) International publication number: WO 2014/192085

(56) References cited:
- JP-A- 2002 163 367
- JP-A- 2008 257 699
- JP-A- 2010 142 562
- KR-A- 20120 042 524

## Description

### Technical Field

The present invention relates to a care robot which assists a care receiver.

### Background Art

As a type of care robot, a care robot disclosed in patent literature 1 is known. As shown in figs. 1 and 3 of patent literature 1, the care robot is a movement assistance device (10) that assists the transfer and movement of a care receiver held using a holding tool (3) provided on an arm section (2), and is provided with an instability measuring means that measures the instability of the care receiver by detecting physical changes linked to the sense of instability of the care receiver, and a control section (17) that performs driving control of driving means (61 to 65) based on instructions entered using an operation section (25) and feedback control to suppress the level of instability measured by the instability measuring means.

The instability measuring means measures, as physical changes linked to the sense of instability of the care receiver, at least one of pulse rate, quantity of perspiration, respiration rate, eye movement, electric skin resistance, and skin temperature which increase as instability increases. The instability measuring means is provided with at least one of a heart rate sensor (40, 41) that measures the pulse rate of the care receiver, a perspiration sensor (42) that measures the quantity of perspiration of the care receiver, a pressure sensor (43) that detects minute vibrations of the care receiver linked to their respiration rate, a camera (44) that images the eye movement of the care receiver, an electric current sensor (45) that detects the electric skin resistance of the care receiver, and a temperature sensor (46) that measures the skin temperature of the care receiver. Further examples of devices that assist a care receiver are disclosed in Patent literature 2 and 3.

### Citation List

### Patent Literature

Patent literature 1: JP-A-4692642
Patent literature 2: JP 2010 142562 A
Patent literature 3: KR 2012 0042524 A

### Summary of Invention

### Technical Problem

In the care robot disclosed in patent literature 1, physical changes of a care receiver (help receiver) held and transferred by a holding tool (3) such as pulse rate are measured directly by a heart rate sensor (40, 41) and so on. Thus, it is possible to monitor directly and in real time the condition of the care receiver who is being held and transferred, and it is possible to appropriately adjust the speed and so on of the arm section (2) based on that condition. However, there are cases in which the condition of the care receiver was not good prior to transfer (assistance), and cases in which, although the condition of the care receiver was not bad prior to transfer, their condition became bad due to being transferred. In these cases, it is not appropriate to transfer the care receiver, or when transferring the care receiver it is necessary to assist the care receiver such that their condition is not made worse.

In order to solve the above problem, an object of the present invention is to provide a care robot that is capable of performing care of a care receiver while more favorably maintaining the body condition of the care receiver.

### Solution to Problem

In order to solve the above problem, the present invention provides a care robot according to independent claim 1. Preferred embodiments are laid down in the dependent claims.

### Brief Description of Drawings

[Fig. 1] Fig. 1 is a schematic diagram illustrating a scheme of a care center in which care robots according to an embodiment of the present invention are arranged.
[Fig. 2] Fig. 2 is a diagram illustrating the relationship between a care robot, terminal device, and host computer.

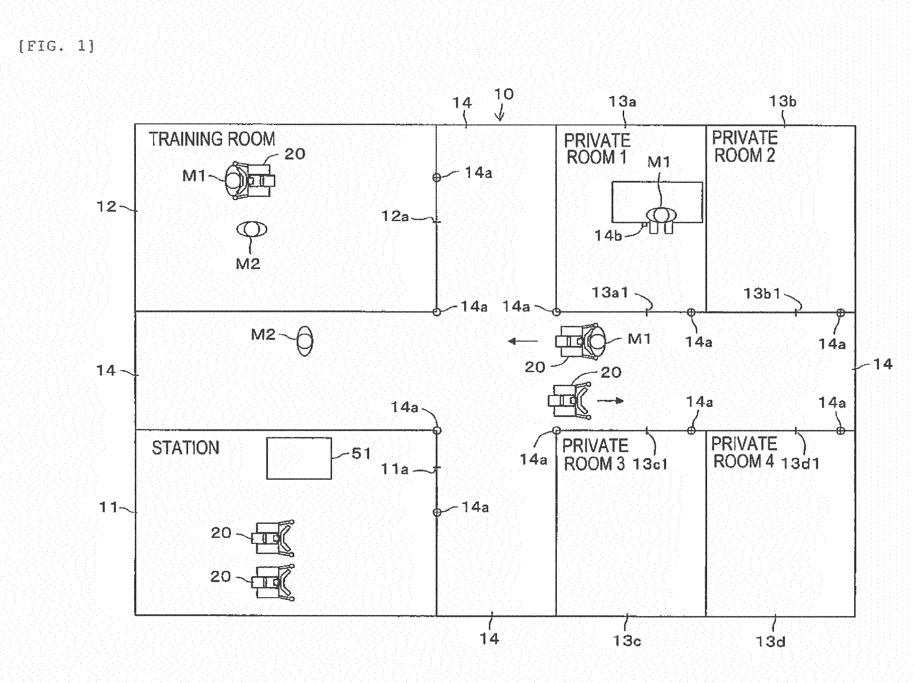

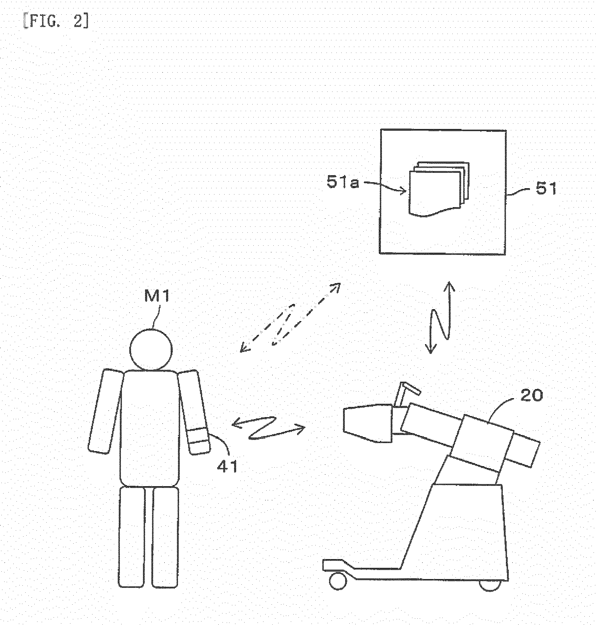

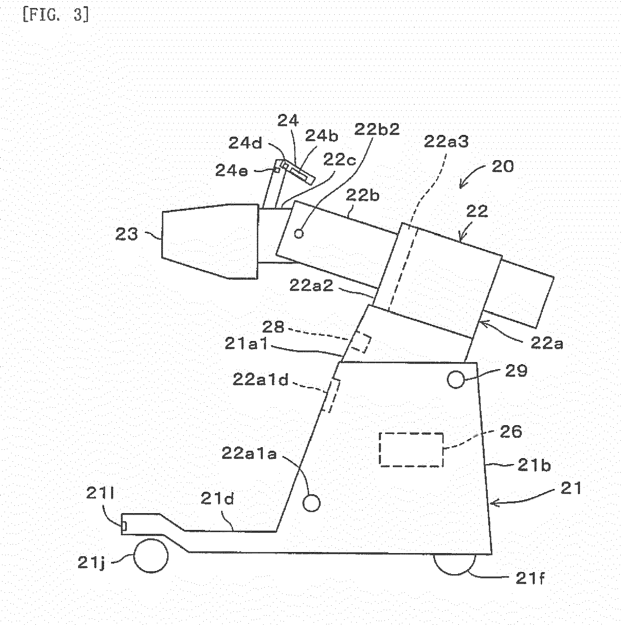

[Fig. 2] Fig. 3 is a right side view illustrating the care robot illustrated in fig. 1.

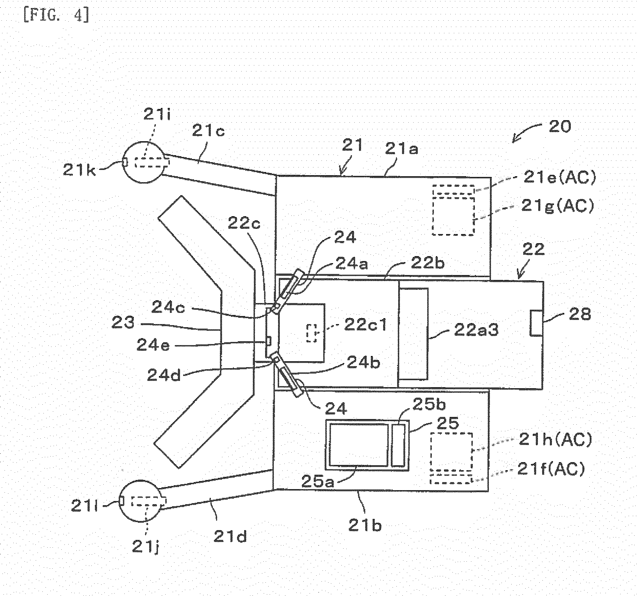

[Fig. 4] Fig. 4 is a plan view illustrating the care robot illustrated in fig. 1.

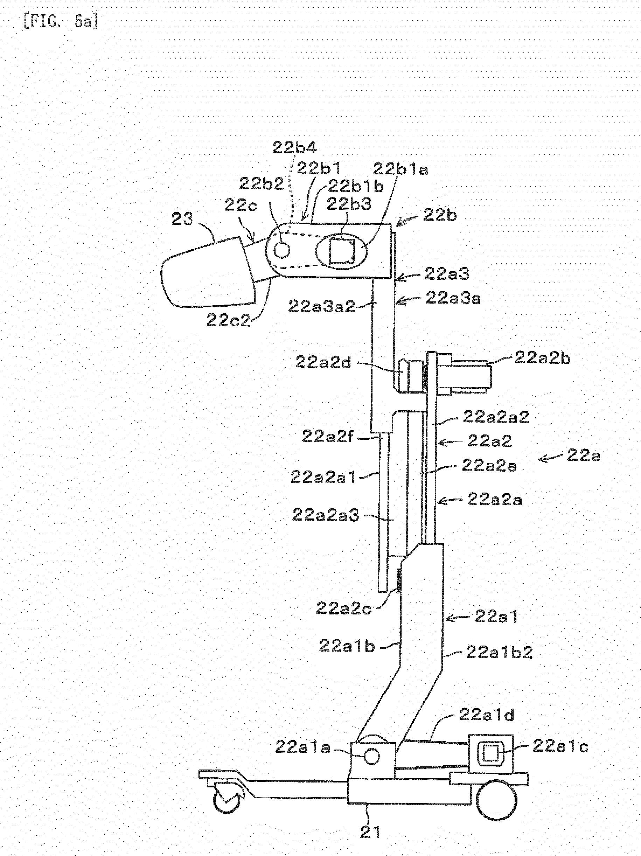

[Fig. 5a] Fig. 5a is a right side view illustrating a scheme of the internal structure of the care robot illustrated in fig. 1 which is in an extended state.

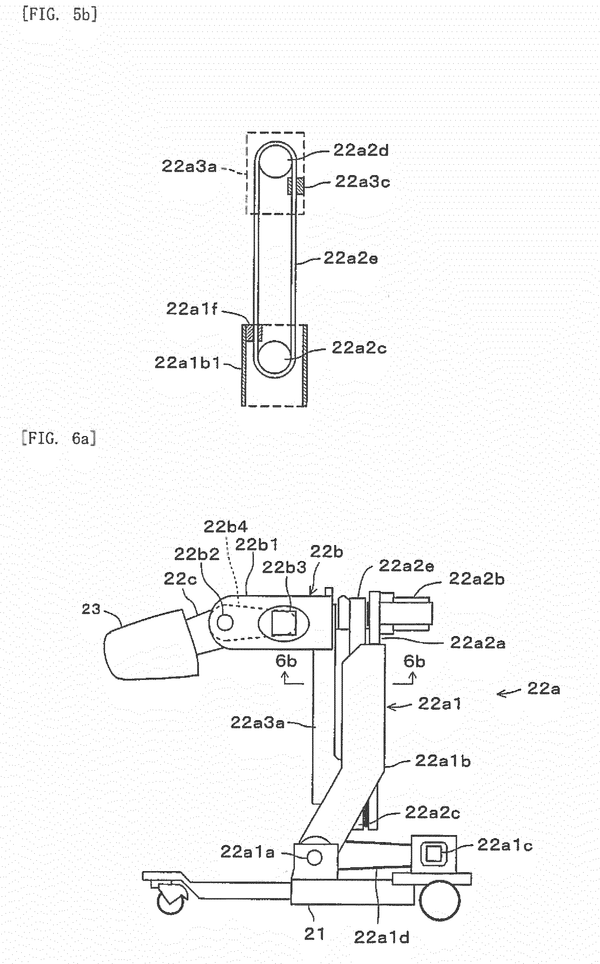

[Fig. 5b] Fig. 5b is a front view illustrating the vicinity including a first slide section illustrated in fig. 5a.
[Fig. 6a] Fig. 6a is a right side view illustrating a scheme of the internal structure of the care robot illustrated in fig. 1 which is in a contracted state.

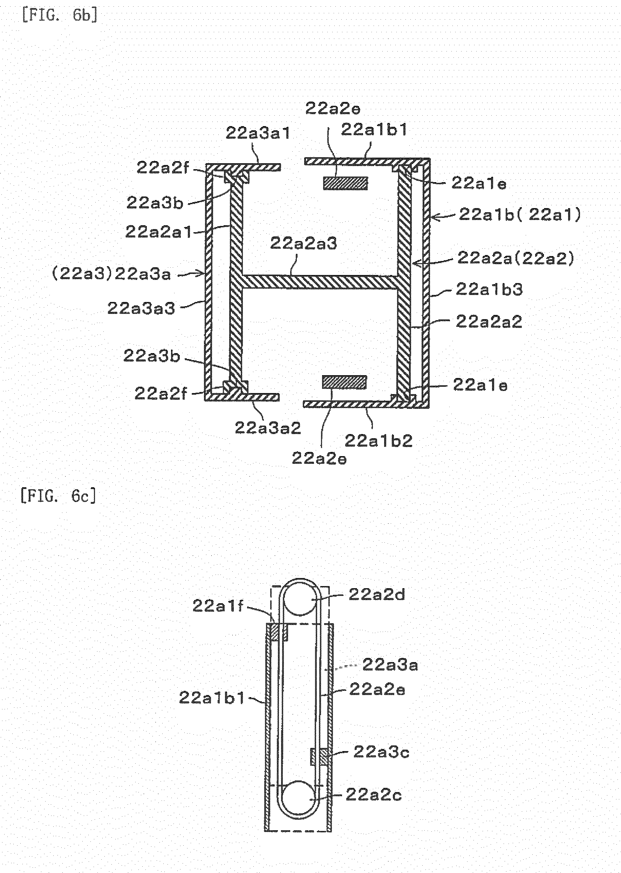

[Fig. 6b] Fig. 6b is an end surface view taken along line 6b-6b illustrated in Fig. 6a.
[Fig. 6c] Fig. 6c is a front view illustrating the vicinity including a first slide section illustrated in fig. 6a.

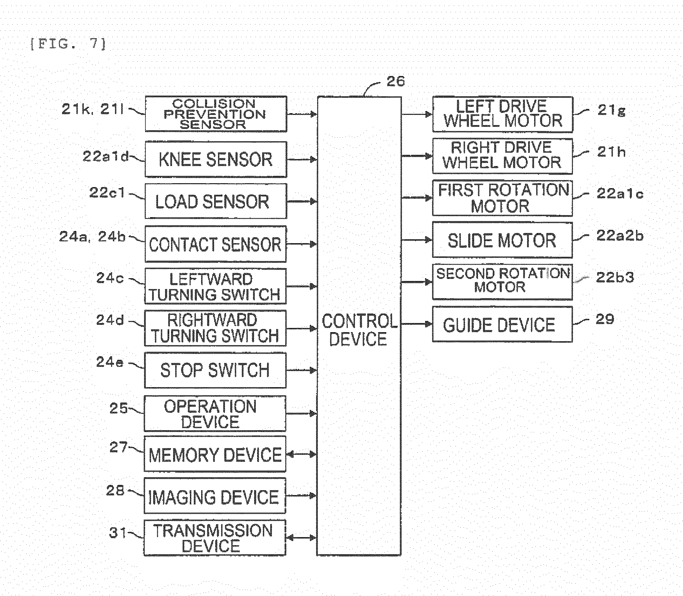

[Fig. 7] Fig. 7 is a block diagram illustrating the care robot illustrated in fig. 1.

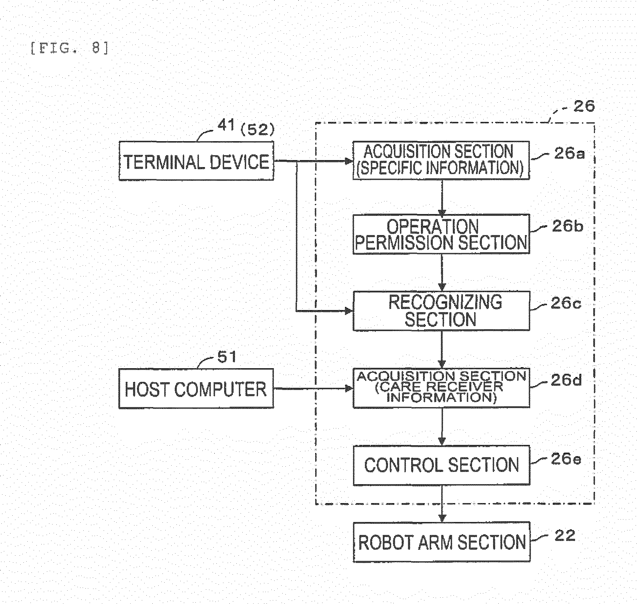

[Fig. 8] Fig. 8 is a block diagram illustrating the control device illustrated in fig. 7.

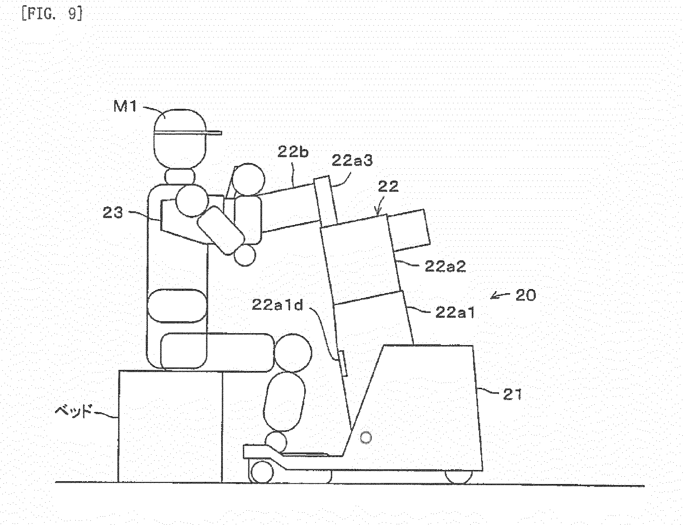

[Fig. 9] Fig. 9 is a side view illustrating a state in which the care robot is supporting a sitting care receiver.

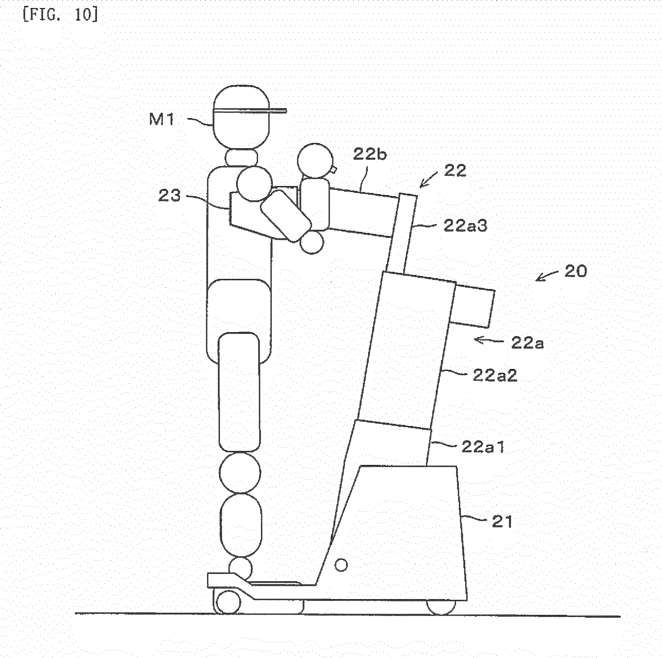

[Fig. 10] Fig. 10 is a side view illustrating a state in which the care robot is supporting a standing care receiver.

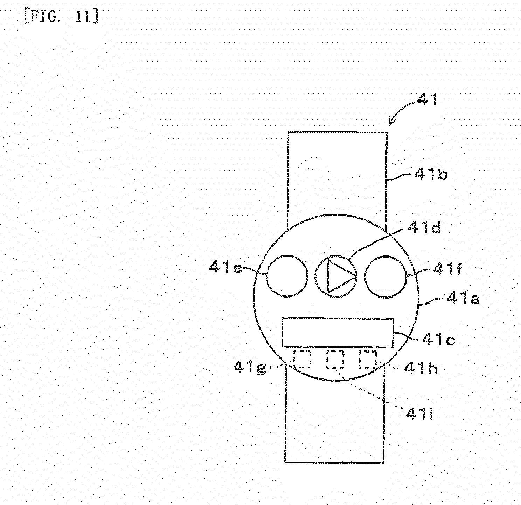

[Fig. 11] Fig. 11 is a top view illustrating a terminal device.

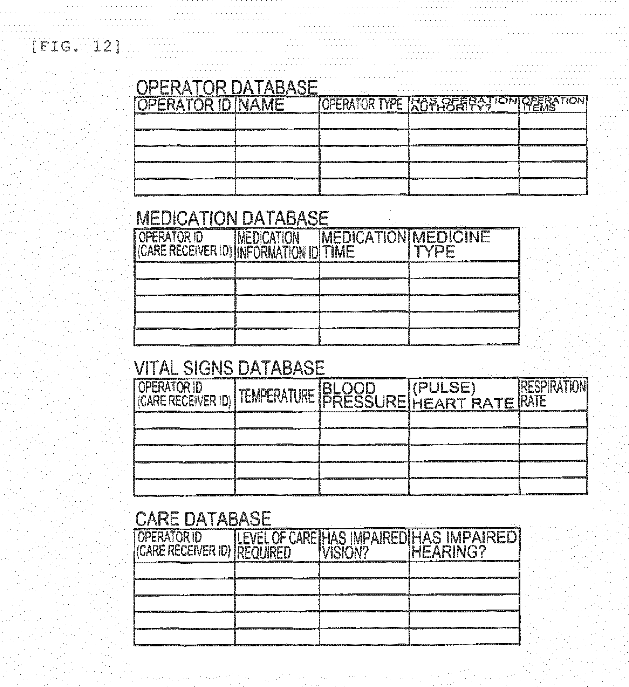

[Fig. 12] Fig. 12 is a diagram illustrating in order from the top an operator database, a medication database, a vital signs database, and a care database.

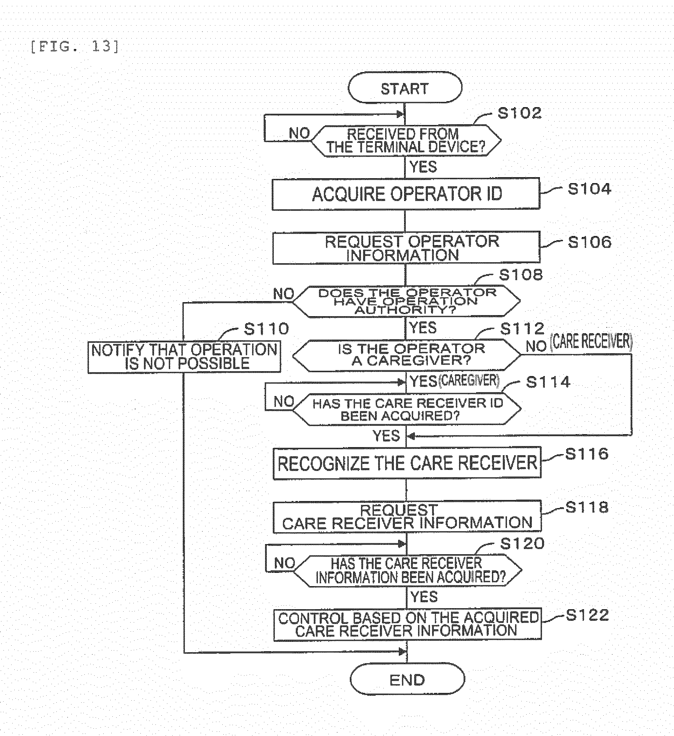

[Fig. 13] Fig. 13 is a flowchart of the operation of the care robot performed by the control device illustrated in fig. 7.

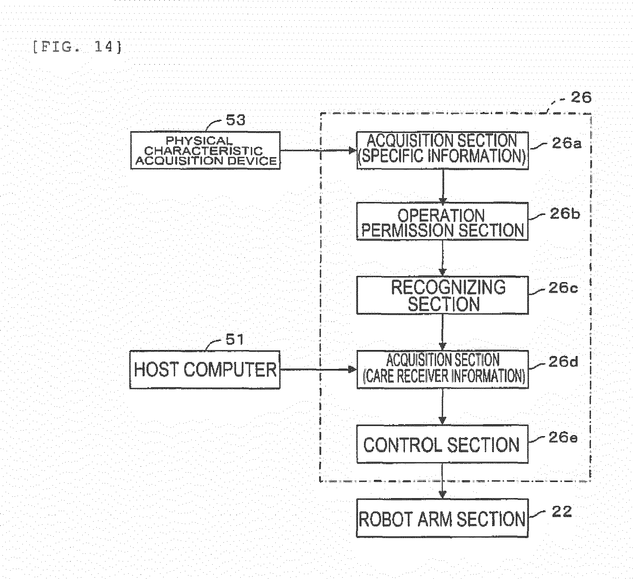

[Fig. 14] Fig. 14 is a block diagram illustrating the control device illustrated in fig. 7 of another embodiment.

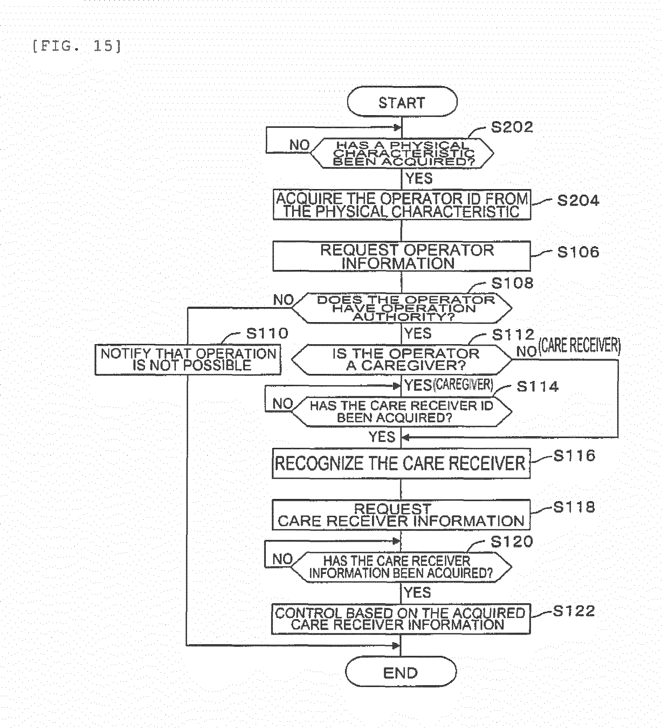

[Fig. 15] Fig. 15 is a flowchart of the operation of the care robot performed by the control device illustrated in fig. 7 of another embodiment.

### Description of Embodiment

Hereinafter, an embodiment of a care robot according to the present invention will be described. Fig. 1 is a schematic view illustrating a scheme of a care center 10 where care robots 20 are arranged. Care center 10 has a station 11, a training room 12, and respective private rooms 13a to 13d. The care center 10 is a residential area where people live. The people in the care center 10 are care receivers M1 who require care and caregivers M2 who assist the care receivers M1.

As shown in fig. 1, station 11 is an office of the caregivers M2, and serves as a base where the care robots 20 are on standby or charged. Training room 12 is a room where the care receivers M1 are in training or rehabilitation. The respective private rooms 13a to 13d are rooms where the care receivers M1 live. Station 11, training room 12, and respective private rooms 13a to 13d have respective entrances/exits 11a, 12a, and 13a1 to 13d1; the respective entrances/exits 11a, 12a, and 13a1 to 13d1 are connected to one another via corridor 14.

Host computer 51 which allows wireless communication between care robots 20 is provided in station 11. Host computer 51 manages treatment/diagnosis information (for example, patient chart information), and care information (for example, rehabilitation information) for care receivers M1, and identification numbers (identification symbols) for care receivers M1 and caregivers M2 all together.

Care robot 20 is allowed to move in the residential area where the persons live, and is moved in the residential area by the movement of left and right drive wheel motors 21g and 21h that serve as drive sources. In fig. 1, an arrow in the vicinity of a care robot 20 indicates the traveling direction of the care robot 20.

As shown in fig. 2, care robot 20 is capable of communication with terminal device 41 worn by a care receiver M1 (or caregiver M2) who is the operator, and is capable of communication with host computer 51. Care robot 20 may also be capable of communication with terminal device 41 via host computer 51.

Care robot 20 is a care robot for assisting the standing up and sitting down of a care receiver M1 by supporting a body part (for example, the upper body, particularly, the chest) of care receiver M1. As shown in figs. 3 and 4, care robot 20 is configured to include base 21, robot arm section 22, holding section 23, handle 24, operation device 25, and control device 26.

Base 21 is provided with left/right base sections 21a and 21b, and left/right leg sections 21c and 21d. Left/right base sections 21a and 21b are provided spaced apart by a predetermined interval in the left/right direction, and as shown in fig. 4, left/right drive wheels 21e and 21f are respectively provided on left/right base sections 21a and 21b, and left/right drive wheel motors 21g and 21h that respectively drive left/right drive wheels 21e and 21f are internally incorporated in left/right base sections 21a and 21b. Care robot 20 travels using left/right drive wheels 21e and 21f which are respectively driven by left/right drive wheel motors 21g and 21h. Traveling drive section AC is configured from left/right drive wheels 21e and 21f, and left/right drive wheel motors 21g and 21h. The configuration may be such that the above drive sources provided in base 21 are omitted and care robot 20 is moved by being pushed by care receiver M1.

Left/right leg sections 21c and 21d extend horizontally in a forward direction (left direction in figs. 3 and 4) from left/right base sections 21a and 21b. Left/right driven wheels 21i and 21j are respectively provided on an end section of left/right leg sections 21c and 21d. In addition, a pair of collision prevention sensors 21k and 21l are respectively provided on an end of the left/right leg sections 21c and 21d. Collision prevention sensors 21k and 21l are sensors for detecting an obstacle, and a detection signal thereof is transmitted to control device 26.

A base section of robot arm section 22 is attached to the base 21 and, as shown in figs. 5a and 6a, robot arm section 22 is provided with multiple arms 22a, 22b, and 22c which are relatively movable by using a drive section configured to mainly include first and second rotation motors 22a1c and 22b3 and a slide motor 22a2b. Robot arm section 22 may be configured from multiple axes. Axes in this case may include at least one of a rotation axis and a slide axis.

As shown in figs. 5a and 5b, and figs. 6a to 6c, a base section of first arm 22a is attached to base 21. First arm 22a is provided with slide base section 22a1, first slide section 22a2, and second slide section 22a3.

As shown in figs. 3 and 4, slide base section 22a1 is formed in a substantially rectangular cuboid shape. As shown in fig. 5a, the base end section of slide base section 22a1 is provided mainly with frame 22a1b attached to base 21 so as to be rotatable around first rotation axis 22a1a. Frame 22a1b is formed with a substantially U-shaped cross section, and as shown in fig. 6b, is configured mainly from left/right plate sections 22a1b1 and 22alb2 which are formed to be bent, and a rear plate section 22a1b3 whose left and right ends are connected to the upper rear ends of the left/right plate sections 22a1b1 and 22a1b2.

As shown in fig. 5a, first rotation motor 22a1c is provided on base 21. First drive belt 22a1d is mounted around a pulley of first rotation motor 22a1c and a pulley of first rotation axis 22ala. When first rotation motor 22a1c is driven, frame 22a1b, that is slide base section 22a1, is rotated around first rotation axis 22a1a in a forward direction or backward direction.

As shown in fig. 6b, a left/right guide groove 22ale that slidably engages with the left/right end of rear plate member 22a2a2 of frame 22a2a of first slide section 22a2 which is described below is formed on the inside of frame 22a1b (on the inside of left/right plate member 22a1b1 and 22a1b2). Fixed section 22alf that is attached to and fixes slide belt 22a2e which is described below is provided on an upper section of left plate member 22a1b1 of frame 22a1b (refer to figs. 5b and 6c).

As shown in figs. 3 and 4, first slide section 22a2 is formed in a substantially rectangular cuboid shape and is configured to be smaller than slide base section 22a1. First slide section 22a2 slides in a lengthwise direction (axis movement direction) with respect to slide base section 22a1, and is configured to be substantially housed inside slide base section 22a1 when contracted.

Specifically, first slide section 22a2 is provided with frame 22a2a (refer to fig. 5a). As shown in fig. 6b, frame 22a2a is formed in an H-shape in cross section and an H-shape in a side view, and is configured from front/rear plate members 22a2a1 and 22a2a2 and a connection plate member 22a2a3 whose front/rear ends are connected to a central portion in the vertical direction of front/rear plate members 22a2a1 and 22a2a2. Both the left and right ends of rear plate member 22a2a2 are slidably engaged with left/right guide groove 22ale of frame 22alb. As shown in fig. 5a, mainly slide motor 22a2b is provided on an upper section of rear plate member 22a2a2. Pulley 22a2c is rotatably provided on a lower section of rear plate member 22a2a2. Slide belt 22a2e is mounted around a pulley 22a2c and a pulley 22a2d of slide motor 22a2b.

As shown in fig. 6b, guide rail 22a2f is provided in both the left and right end sections of the front plate member 22a2a1 of frame 22a2a. Guide rail 22a2f slidably engages with left/right guide receiving section 22a3b on the inside of the left/right plate members of frame 22a3a of second slide section 22a3 which is described below.

As shown in figs. 3 and 4, second slide section 22a3 is formed in a substantially rectangular cuboid shape and is configured to be smaller than first slide section 22a2. Second slide section 22a3 slides in a lengthwise direction (axis movement direction) with respect to first slide section 22a2, and is configured to be substantially housed inside first slide section 22a2 when contracted.

Specifically, second slide section 22a3 is provided with frame 22a3a (refer to fig. 5a). As shown in fig. 6b, frame 22a3a is formed substantially in an H-shape in cross section and an H-shape in a side view, and is configured from left/right plate members 22a3a1 and 22a3a2 and a front/rear plate member 22a3a3 whose left/right ends are connected to the front section of left/right plate members 22a3a1 and 22a3a2. Left/right guide receiving section 22a3b that slidably engages with guide rail 22a2f of frame 22a2a is provided on the inside of frame 22a3a (the inner wall of left/right plate members 22a3a1 and 22a3a2). Fixed section 22a3c that is attached to and fixes slide belt 22a2e is provided on a lower section of right plate member 22a3a2 of frame 22a3a (refer to figs. 5b and 6c).

If slide motor 22a2b is driven, frame 22a2a of first slide section 22a2 extends in the axis movement direction with respect to frame 22a1b of slide base section 22a1. At the same time, frame 22a3a of second slide section 22a3 extends with respect to frame 22a2a of first slide section 22a2 (the extended state shown in figs. 5a and 5b).

Conversely, if slide motor 22a2b is driven in the reverse direction, frame 22a2a of first slide section 22a2 contracts in the axis movement direction with respect to frame 22a1b of slide base section 22a1 (the contracted state shown in figs. 6a and 6c). At the same time, frame 22a3a of second slide section 22a3 contracts with respect to frame 22a2a of first slide section 22a2 (the contracted state shown in figs. 6a and 6c).

As shown in figs. 3 and 4, second arm 22b is formed in a substantially rectangular cuboid shape and is formed in an end section of second slide section 22a3 extending in a direction (forward direction) that is perpendicular to the lengthwise direction. Specifically, as shown in fig. 5a, second arm 22b is mainly provided with frame 22b1 configured from left/right plate members 22b1a and 22b1b. The rear end of left/right plate members 22b1a and 22b1b of frame 22b1 are respectively fixedly connected to an upper section of left/right plate members 22a3a1 and 22a3a2 of frame 22a3a.

Second rotation axis 22b2 is rotatably provided on an end section of left/right plate members 22b1a and 22b1b of frame 22b1. Second rotation motor 22b3 is provided on a central section of left/right plate members 22b1a and 22b1b. Second rotation drive belt 22b4 is mounted around a pulley of second rotation motor 22b3 and a pulley of second rotation axis 22b2.

Third arm 22c is formed in a substantially rectangular cuboid shape and the base section thereof is attached to an end section of second arm 22b so as to be rotatable around second rotation axis 22b2. Specifically, third arm 22c is provided with frame 22c2. The rear end section of frame 22c2 is fixed so as to be rotated as one with second rotation axis 22b2. The front end section of frame 22c2 is fixed to the rear end of holding section 23.
If second rotation motor 22b3 is driven, frame 22c2, that is third arm 22c, rotates around second rotation axis 22b2 in an upward direction or a downward direction.

Holding section 23 is fixed to an end of third arm 22c. Holding section 23 assists the standing up and sitting down of care receiver M1 by supporting a body part (for example, the upper body, particularly, the chest) of care receiver M1. For example, holding section 23 is a member that supports both arms (both armpits) of care receiver M1 from below when working opposite care receiver M1 during standing up motion and sitting down motion, and is formed in a U-shape which is open in the forward direction in a plan view. Holding section 23 is formed, for example, from a relatively soft material on the assumption that holding section 23 contacts care receiver M1.

As shown in figs. 3 and 4, handle 24 is fixed to the upper surface of third arm 22c. Handle 24 is configured from a pair of left and right rod-shaped handgrips, and is provided such that the handgrips are to be gripped by the left and right hands of care receiver M1. Contact sensors 24a and 24b for detecting the grip are provided in handle 24. Leftward turning switch 24c for turning the care robot 20 to the left and rightward turning switch 24d for turning the care robot 20 to the right are provided in handle 24. Furthermore, stop switch 24e for stopping the care robot 20 is provided in handle 24.

In addition, in a case where care receiver M1 walks in a state being supported by holding section 23, or in a case where care receiver M1 walks in a state gripping handle 24, load sensor 22c1 for detecting the force received from care receiver M1 is provided in third arm 22c. Load sensor 22c1 is a sensor for detecting as a voltage change the distortion amount of a distortion generating body which changes according to a load change, or a semiconductor-type pressure sensor in which gauge resistance is changed and converted into an electrical signal according to the distortion which arises when pressure is applied to a silicon chip.

Operation device 25 is provided with display unit 25a that displays images and operation section 25b that receives input operations from an operator (caregiver M2 or care receiver M1).
Display section 25a is configured from a liquid crystal display, and displays a selection screen for operation modes of the care robot 20 and so on. A standing up motion assistance mode for assisting a standing up motion of care receiver M1, a sitting down motion assistance mode for assisting a sitting motion care receiver M1, and so on, are set as operation modes. Also, modes which correspond to body parts that caregiver M1 wants to train are set within the standing up assistance mode. For these modes there are, for example, an upper body mode for training the upper body, particularly, the back muscles, and a lower body mode for training the lower body, particularly, the legs.

Operation section 25b is provided with a cursor key for moving a cursor up/down/left/right, a cancel key for canceling inputs, and a determination key for determining selected content; the configuration is such that operator instructions can be entered using the keys. Operation unit 25 may be configured from a touch panel that has the display function of display section 25a and the input function of operation section 25b such that the device is operated by pressing the display on the touch panel.

Storage device 27 (storage section) stores: standing up trajectory reference data that indicates a standing up trajectory along which a movement control position, for example a shoulder position, of care receiver M1 passes when a sitting care receiver M1 (refer to fig. 9) who is supported by holding section 23 stands up; and sitting down trajectory reference data that indicates a sitting down trajectory, which is different from the standing up trajectory, along which a shoulder position of care receiver M1 passes when a standing care receiver M1 (refer to fig. 10) who is supported by holding section 23 sits down.

Also, storage device 27 may store multiple different standing up trajectory data for training multiple different body parts of care receiver M1. Note that the above respective trajectory data is expressed as two-dimensional coordinates or robot coordinates.

Further, storage device 27 stores a correction amount (first correction amount) according to the height of a seat section such as a chair or a bed on which care receiver M1 sits, and a correction amount (second correction amount) according to the height of care receiver M1. These first and second correction amounts are values for correcting the above data.

Specific information acquisition device 52 acquires specific information for identifying care receiver M1, and in the present embodiment includes terminal device 41, and receiver 31 that receives transmitted data from terminal device 41. Specific information is the identification number (operator ID, care receiver ID) of care receiver M1 and care caregiver M2.

Control device 26 performs control related to traveling and stance changing of the care robot 20. As shown in fig. 7, the above collision prevention sensors 21k and 21l, knee sensor 22a1d, load sensor 22c1, contact sensors 24a and 24b, leftward turning switch 24c, rightward turning switch 24d, stop switch 24e, left and right drive wheel motors 21g and 21h, first rotation motor 22alc, slide motor 22a2b, second rotation motor 22b3, operation device 25, storage device 27, imaging device 28, guide device 29, and transceiver 31 are connected to the control device 26. Also, control device 26 has a microcomputer (not shown); the microcomputer is provided with an input/output interface, CPU, RAM, and ROM (all not shown) that are connected to one another via a bus.

As shown in fig. 8, control device 26 is provided with specific information acquisition section 26a, operation permission section 26b, recognizing section 26c, care receiver information acquisition section 26d, and control section 26e. Specific information acquisition section 26a acquires specific information acquired by specific information acquisition device 52 from specific information acquisition device 52. Operation permission section 26b allows operation of care robot 20 when the operator has operation authority for the operation, and prohibits operation of care robot 20 when the operator does not have operation authority for the operation. Recognizing section 26c recognizes care receiver M1 from the specific information acquired by specific information acquisition device 52. Care receiver information acquisition section 26d acquires information related to care receiver M1 recognized by recognizing section 26c from host computer 51. Control section 26e performs drive control of holding section 23 by driving robot arm section 22 based on the information related to care receiver M1 acquired by care receiver information acquisition section 26d.

Imaging device 28 is provided respectively on the front surface of slide base section 22a1 and the rear surface of first slide section 22a2. Imaging device 28 provided on the front surface of slide base section 22a1 images a target in front of moving body 20. Imaging device 28 provided on the rear surface of first slide section 22a2 images a target behind or above moving body 20.

Guide device 29 provides guidance to people, including care receiver M1 and caregiver M2, in the vicinity regarding the state of the care robot 20 using sound or a display. Guide device 29 may be a speaker for outputting sound, or a display device such as an LCD or an LED for displaying characters or graphics and the like.

Transceiver 31 is capable of communication with the transceiver of terminal device 41 and the transceiver of host computer 51. Transceiver 31 receives transmission data from terminal device 41 and host computer 51, and sends transmission data to terminal device 41 and host computer 51.

Terminal device 41 is for performing remote operation of care robot 20. Terminal device 41 is a terminal device that is worn by care receiver M1, has an identification symbol which is linked to care receiver M1, and sends operation instructions for care robot 20 and the identification symbol of care receiver M1 as transmission data.

As shown in fig. 11, terminal device 41 is a wristwatch type worn on the wrist of an operator and is provided with main body 41a formed in a disk shape and band 41b connected at both ends. Main body 41a is provided with display section 41c, start switch 41d, menu switch 41e, selection switch 41f, transceiver 41g, GPS receiver 41h, and control device 41i.

Display device 41c displays the operating state (for example, recognizing, on standby, standing up, sitting down, and so on) of care robot 20, the result of operation permission (for example, "No operation authority"), and the like. Start switch 41d is a switch for starting operation of care robot 20. Start switch 41d also functions as a determination switch.

Menu switch 41e is a switch for displaying a menu screen which is a screen for displaying an operation mode on display device 41c. A standing up motion assistance mode for assisting a standing up motion of a user, a sitting down motion assistance mode for assisting a sitting down motion of a user, and a movement assistance mode for assisting a movement of a user are set as operation modes. A standing up walking assistance mode, an elbow support walking assistance mode, a hand support walking assistance mode, a standing up riding movement mode, and a seated riding movement mode are set as movement assistance modes. Selection switch 41f is a switch for selecting a desired menu item (operation mode) from the menu screen.

Transceiver 41g is capable of communicating with transceiver 31 of care robot 20. Transceiver 41g may be capable of communicating with host computer 51. GPS receiver 41h receives a signal from a GPS for deriving the current position thereof. Control device 41i is connected to display section 41c, start switch 41d, menu switch 41e, selection switch 41f, transceiver 41g, and GPS receiver 41h, and controls them.

The storage section of control device 41i stores an operator ID which is an identification number (identification symbol) linked to the operator. Note that, when terminal device 41 is worn by a new operator, it is preferable for the ID of that operator to be written to the storage section of control device 41i.

Host computer 51 manages treatment/diagnosis information for care receivers M1 (for example, patient chart information), care information (for example, rehabilitation information), and identification numbers (identification symbols) for care receivers M1 and caregivers M2 all together. Database 51a such as those shown in fig. 12 is stored in host computer 51. Database 51a is, for example, an operator database, a medication database, a vital signs database, a care database, and so on.

An operator database is configured from operator information such as operator ID, operator name, operator type (care receiver or caregiver), existence of operation authority, operation items, and so on. A medication database is configured from medication information such as operator ID (care receiver ID), medication information ID which is an identification number of medication information, medication time, medicine type, and so on. A vital signs database is configured from vital signs information such as operator ID (care receiver ID), and vital signs information such as the temperature, blood pressure, pulse (heart rate), respiration rate and so on of the care receiver. Note that, vital signs information may include trends (change amounts) of these vital signs. A care database is configured from care information such as operator ID (care receiver ID), care receiver level of care required, presence (extent) of impaired vision, presence (extent) of impaired hearing, and so on.

This database 51a is updated with the latest information. In other words, after diagnosis, treatment, and rehabilitation, information related to care receiver M1 with regard to these is sent to host computer 51, and host computer 51 updates the information related to care receiver M1 based on this information.

Next, an example of operation of care robot 20 as configured above will be described using the flowchart shown in fig. 13.
In step S102, control device 26 decides whether transmission data (including an operation start signal) has been received from terminal device 41. For example, in a case in which an operator has pushed start switch 41d of terminal device 41, terminal device 41 sends transmission data including an operation start signal, operator ID, and current position to care robot 20.

At this time, control device 26 receives transmission date from terminal device 41, so determines "YES" in step S102 and continues the program to step S104. Note that in cases in which there is no transmission data from terminal device 41, control device 26 repeats the determination of "NO" in step S102.

In step S104, control device 26 acquires an operator ID from transceiver 31. Then, in step S106, control device 26 requests host computer 51 for the operator information corresponding to the operator ID just acquired. Host computer 51 sends the corresponding operator information, that is, operator name, operator type (care receiver or caregiver), existence of operation authority, and operation items to the requesting care robot 20. Then, control device 26 receives (acquires) the operator information corresponding to the operator ID just acquired.

In step S108, control device 26 determines whether the operator has operation authority based on the operator information acquired in step S106. If the operator has operation authority, in step S108, control device 26 determines "YES" and continues the program to step S112. Conversely, if the operator does not have operation authority, in step S108, control device 26 determines "NO" and continues the program to step S110.

In step S110, control device 26 gives a notice that "operation is not possible". Specifically, control device 26 displays "Operation is not possible" on display section 25a, or guides by playing a sound from guide device 29. Control device 26 also sends an indication "Operation is not possible" to terminal device 41. Terminal device 41 displays "Operation is not possible" on display section 41c. Further, control device 26 prohibits (restricts) operation of care robot 20.

In step S112, control device 26 determines whether the operator is a caregiver or a care receiver based on the operator information acquired in step S106. If the "operator type" is "caregiver", control device 26 determines "YES" in step S112 and continues the program to step S114. Conversely, if the "operator type" is "care receiver", control device 26 determines "NO" in step S112 and continues the program to step S116.

In a case in which the operator is caregiver M2, because the target for care of care robot 20, that is, the care receiver, cannot be known specifically, control device 26 waits for the care receiver ID (operator ID) which is the identification number of care receiver M1 or the name of care receiver M1 to be inputted (the "NO" determination of step S114 is repeated). When caregiver M2 inputs the care receiver ID (or name) using operation section 25b of operation device 25, or when caregiver M2 inputs the care receiver ID by operating menu switch 41e and selection switch 41f of terminal device 41, control device 26 determines "YES" in step S114.

In step S116, control device 26 recognizes care receiver M1 from the care receiver ID. Thus, care receiver M1 who is the target of care of care robot 20 is identified. Accordingly, in step S118, control device 26 requests information related to the care receiver corresponding to the care receiver ID just acquired from host computer 51. Host computer 51 sends information related to the corresponding care receiver, for example, medication information such as medication information ID which is the identification number of the medication information, medication time, and medication type, vital signs information such as temperature, blood pressure, pulse, respiration rate, and care information such as level of care required, presence (extent) of impaired vision, presence (extent) of impaired hearing, to the requesting care robot 20.

In step S120, control device 26 determines whether information related to the care receiver has been acquired. Control device 26 repeats the determination of "NO" in step S120 until information related to the care receiver is received; when the information related to the care receiver is received, control device 26 determines "YES" in step S120 and continues the program to step S122.

In step S122, control device 26 controls care robot 20 based on the information related to the care receiver just acquired in step S118. For example, as shown in fig. 1, a case in which a care receiver M1 who is in first private room 13a calls a care robot 20 will be described. As given above, care receiver M1 can call a care robot 20 to their current position in first private room 13a by pushing start switch 41d of terminal device 41.

First, movement of care robot 20 is described. A case is described in which care robot 20 moves independently from station 11 to the respective private rooms 13a to 13d (or from the respective private rooms 13a to 13d to station 11). When moving through corridor 14 from station 11 to the respective private rooms 13a to 13d, care robot 20 moves along a route stored in advance in storage device 27 which is a route from the entrance/exit of station 11 to the respective entrance/exit of the respective private rooms 13a to 13d.

Also, care robot 20 reads guiding marks 14a provided in corridor 14 using imaging device 28, calculates the remaining traveling distance from the information, and moves based on the calculation result. For example, guiding marks 14a may be two-dimensional barcodes. Information such as current position (for example, an intersection of corridor 14), and distance and direction from the current position to the target position (for example, in a case in which care robot 20 moves from station 11 to first private room 13a, when the care robot 20 approaches an intersection of corridor 14, the distance and direction [left turn] from the intersection to the first private room 13a) is stored in the two-dimensional barcode. Guiding marks 14a are provided at entrance/exit 11a of station 11, the corner of the respective entrance/exit 13a1 to 13d1 of respective private rooms 13a to 13d, and specified locations in corridor 14 (for example, corners of intersections, or the ceiling).

Next, a case will be described in which care robot 20 comes close to a sitting care receiver M1. In this case, care robot 20 enters first private room 13a through entrance/exit 13a1 of first private room 13a and then approaches the care receiver M1, who is sitting on the edge of the bed. Care robot 20 advances with the front surface of care robot 20 facing in the traveling direction. Care robot 20 reads a guiding mark 14b provided in the vicinity of care receiver M1 using imaging device 28 in the front surface of care robot 20 (or receives the current position information from terminal device 41), and approaches care receiver M1 based on the information.

Furthermore, a standing up operation and a sitting down operation of care robot 20 will be described with reference to figs. 9 and 10. Care robot 20 uses a detection result (distance between the care robot 20 and a knee of the care receiver M1) of knee sensor 22a1d, and moves to a predetermined position where the distance from a sitting care receiver M1 becomes a predetermined distance. The predetermined position is the optimum position for allowing care receiver M1 to stand up (stand up optimum position).

Then, care robot 20 gives the guidance "Grip the handle" to care receiver M1 using guide device 29. If care receiver M1 grips both handles, the fact that handle 24 has been gripped is detected by contact sensors 24a and 24b, thus care robot 20 performs standing up operation for allowing the care receiver M1 to stand up.

During standing up operation, care robot 20 is controlled based on the information related to the care receiver just acquired in step S118. For example, robot arm 22 or holding device 23 is controlled at a standing up operation speed according to the time from taking medication until care is performed, such as in a case in which 30 minutes or fewer have elapsed since medication was taken, standing operation speed is made slower than usual and so on. Also, robot arm 22 or holding device 23 may be controlled at a standing up operation speed according to vital signs of care receiver M1 measured in advance such as temperature, blood pressure, pulse, respiration rate and so on, or the trends (change amount) thereof. Note that, the assistance amount of robot arm 22 or holding section 23 may be adjusted according the time from taking medicine until care is performed or the vital signs. Or, it may be adjusted according to both. Also, the same applies during sitting down operation described below.

If a standing up operation starts, care robot 20 holds the upper body of sitting care receiver M1 using holding section 23 (refer to fig. 9). Then, while holding the upper body of care receiver M1, care robot 20 stands the care receiver M1 up (refer to fig. 10).

Care robot 20 assists care receiver M1 in a standing up state. Care receiver M1 moves by walking in a state supported by holding section 23 supporting the arms of care receiver M1 from below. In a case in which care robot 20 assisting the walking of care receiver M1 in this way moves from first private room 13a to training room 12, similarly to the above-described case in which care robot 20 moves independently, care robot 20 moves along a route stored in advance, or moves by reading guiding marks 14a using imaging device 28.

During this movement, in a case in which there is an area which care receiver M1 is prohibited to enter, even if care receiver M1 tries to enter that prohibited area, care robot 20 prevents entry and guides the care receiver to training room 12 which is the original target location. Entry prohibited areas are one of the pieces of information related to a care receiver.

Care robot 20 turns right at entrance/exit 13a1 of first private room 13a, enters corridor 14, turns right at the intersection of corridors 14, turns left at entrance/exit 12a of training room 12, and enters training room 12. Care robot 20 advances with the rear surface of care robot 20 facing in the traveling direction.

Also, if a sitting down operation for sitting care receiver M1 down starts, care robot 20 brings care receiver M1 in a standing up state (refer to fig. 10) into a sitting down state while the upper body of care receiver M1 is held by the holding section 23 (refer to fig. 9).

Then, when the sitting down operation ends, care robot 20 gives the guidance "Let go of the handle" to care receiver M1 using guide device 29. If care receiver M1 lets go of handle 24, contact sensors 24a and 24b detect the fact that the hands have let go of handle 24, thus, care robot 20 moves away from care receiver M1.

As given above, the present embodiment of a care robot 20 is provided with: holding section 23 provided on base 21 that assists with standing up and sitting down by supporting a body part of care receiver M1; specific information acquisition device 52 that acquires specific information (care receiver ID) for identifying care receiver M1; a recognizing section (control device 26: step S116) that recognizes care receiver M1 from the specific information acquired by specific information acquisition device 52; an acquisition section (control device 26: step S118) that acquires information related to care receiver M1 recognized by the recognizing section; and a control section (control device 26: step S122) that performs drive control of holding section 23 based on the information related to care receiver M1 acquired by the acquisition section.

Accordingly, by recognizing care receiver M1 from the specific information (care receiver ID) acquired by specific information acquisition device 52 that acquires specific information (care receiver ID) for identifying care receiver M1, the target for care of care robot 20 can be identified accurately and reliably without mistake. Further, information (medication information, vital signs information) related to care receiver M1 specified in advance can be acquired in advance and accurately before care robot 20 assists care receiver M1. Then, when care robot 20 performs assistance, it is possible to perform appropriate drive control of holding section 23 according to information (medication information, vital signs information) related to care receiver M1 acquired in advance. Because assistance of care receiver M1 can be performed based on pre-assistance previous information (medication information, vital signs information) related to care receiver M1 in this way, it is possible to perform assistance of care receiver M1 while more favorably maintaining the body condition of care receiver M1.

Also, specific information acquisition device 52 comprises terminal device 41 that is worn by care receiver M1, has an identification symbol (care receiver ID) linked to care receiver M1, and sends operation instructions for care robot 20 and an identification symbol (care receiver ID) of care receiver M1 as transmission data, and receiver 31 that receives transmission data from terminal device 41; and the recognizing section (control device 26: step S116) recognizes care receiver M1 from the identification symbol (care receiver ID) of care receiver M1 which is among the transmission data received by receiver 31.

Accordingly, by care receiver M1 wearing terminal device 41 and the identification symbol (care receiver ID) of care receiver M1 being received from that terminal device 41, care receiver M1 who is the target of care for care robot 20 can be identified. That is, the care receiver M1 whom care robot 20 is to assist can be identified accurately with a simple configuration.

Also, specific information acquisition device 52 comprises terminal device 41 that is worn by caregiver M2 who assists care receiver M1, uses an identification symbol linked to caregiver M2, and sends operation instructions for care robot 20, an identification symbol of caregiver M2 (operator ID: caregiver ID), and an identification symbol (care receiver ID) of care receiver M1 as transmission data, and receiver 31 that receives transmission data from terminal device 41; the recognizing section (control device 26: step S116) recognizes care receiver M1 from the identification symbol (care receiver ID) of care receiver M1 which is among the transmission data received by receiver 31.

Accordingly, in a case in which the operator is caregiver M2, the identification symbol (operator ID: caregiver ID) of caregiver M2 is received from terminal device 41 worn by caregiver M2 and the operator is recognized as caregiver M2; then care receiver M1 who is the target for care for care robot 20 can be identified by the identification symbol (care receiver ID) of care receiver M1 being inputted by that caregiver M2. That is, even in a case in which the operator is caregiver M2, the care receiver M1 whom care robot 20 is to assist can be identified accurately with a simple configuration.

Also, further provided is an operation permission section (control device 26: steps S108, 110, 112) which, in cases in which care receiver M1 and caregiver M2 are operators who operate care robot 20, permits operation when the operator has operation authority with respect to the operation of care robot 20, and prohibits operation when the operator does not have operation authority.

Accordingly, care robot 20, after identifying an operator, can allow/prohibit (restrict the operation contents) an operation that the operator requests based on the presence/absence (authority contents) of authority of the identified operator. Accordingly, by setting operation authority to "none" in cases in which, for example, care receiver M1 suffers from dementia, mistaken operation by care receiver M1 suffering from dementia can be prevented, and appropriate assistance can be given to that care receiver M1.

Note that, in the above embodiment, in a case in which the operator is care receiver M1, operation authority is given, but there are cases in which the operation contents are restricted. For example, depending on the degree of dementia, prohibiting the calling of care robot 20 from a remote location.

Also, the control section (control device 26: step S122) performs adjustment of at least one of speed and assistance amount of holding section 23 when assisting care receiver M1 to stand up or sit down, based on information related to care receiver M1 acquired by the acquisition section (control device 26: step S118).

Accordingly, care robot 20, after identifying care receiver M1, can perform appropriate assistance according to the information (for example, care receiver body condition information [vital signs information] prior to assistance from the care robot) related to that care receiver M1.

Also, care robot 20 is further provided with traveling drive section AC which moves base 21. Accordingly, care robot 20 can provide appropriate walking assistance when assisting the walking of care receiver M1.

Also, with care robot 20, the control section (control device 26: step S122), when assisting the movement of care receiver M1, performs adjustment of the moving speed of base 21 by controlling traveling drive section AC, based on information related to care receiver M1 acquired by the acquisition section (control device 26: step S118).

Accordingly, care robot 20, after identifying care receiver M1, can perform appropriate walking assistance according to the information (for example, care receiver body condition information [vital signs information] prior to assistance from the care robot) related to that care receiver M1.

Note that, in the above embodiment, in a case in which care receiver M1 has a visual impairment it is preferable to give guidance via sound rather than display; in a case in which care receiver M1 has a hearing impairment it is preferable to give guidance via display rather than sound. Whether a care receiver has a visual impairment or whether a care receiver has a hearing impairment may be acquired from care information in the care database.

Also, in the above embodiment, transmission data is received initially by care robot 20 from terminal device 41, however, transmission data may be received by host computer 51 directly from terminal device 41, and then may be sent together with the care receiver ID and information related to the care receiver to care robot 20.

Further, another embodiment of care robot 20 configured as described above will be described with reference to the block diagram shown in fig. 14 and the flowchart shown in fig. 15. In this embodiment, specific information acquisition device 53 differs from the above embodiment in that it is a physical characteristic acquisition device that acquires a physical characteristic of care receiver M1. The physical characteristic acquisition device is a device that reads a physical characteristic such as a fingerprint, voiceprint, retina, or vein pattern on the palm of the hand. This physical characteristic is specific information of a care receiver. Specific information acquisition device 53 may be provided on operation device 25 of care robot 20 or on terminal device 41.

This embodiment is basically the same as the flowchart shown in fig. 13, differences are described with reference to fig. 15, contents which are the same use the same symbols and descriptions thereof are omitted.

In step S202, control device 26 determines whether a physical characteristic has been acquired from specific information acquisition device 53. For example, in a case in which a physical characteristic such as a fingerprint is read by specific information acquisition device 53 when an operator operates operation device 25, specific information acquisition device 53 acquires the physical characteristic data.

At this time, in a case in which physical characteristic data has been acquired, control device 26 determines "YES" in step S202 and continues the program to step S204. Note that, in a case in which physical characteristic data has not been acquired, control device 26 repeatedly determines "NO" in step S202.

In step S202, control device 26 acquires an operator ID from the acquired physical characteristic data. At this time, control device 26 may receive the operator ID by sending the acquired physical characteristic data to host computer 51 and referencing the physical characteristic database in host computer 51. Then, in step S106, control device 26 requests host computer 51 for the operator information corresponding to the operator ID just acquired.

According to this embodiment, specific information acquisition device 53 is a physical characteristic acquisition device that acquires a physical characteristic of care receiver M1; the recognizing section (control device 26: step S116) recognizes the care receiver from the physical characteristic acquired by the physical characteristic acquisition device.

Accordingly, care robot 20 can identify care receiver M1 who is the target for care from the physical characteristic of care receiver M1 acquired by physical characteristic acquisition device 53. That is, the care receiver M1 whom care robot 20 is to assist can be identified accurately with a simple configuration.

Note that, in the above embodiments, processing from step S102 (step S202) to step S118 may be performed in host computer 51.

### Reference Signs List

10: care center; 11: station; 12: training room; 13a to 13d: first to fourth private rooms; 14: corridor; 20: care robot; 21: base; 21g, 21h: left/right drive wheel motor (traveling drive section); 22: robot arm section; 22a: first arm; 22a1c: first rotation motor (drive section); 22a2b: slide motor (drive section); 22b: second arm; 22b3: second rotation motor (drive section); 22c: third arm; 23: holding section; 24: handle; 25: operation device; 26: control device (recognizing section, acquisition section, control section, operation permission section); 26a: acquisition section; 26b: operation permission section; 26c: recognizing section; 26d: acquisition section; 26e: control section; 27: storage device; 28: imaging device; 29: guide device; 31: transceiver (receiver); 41: terminal device; 51: host computer; 52, 53: specific information acquisition device; M1: care receiver; M2: caregiver

## Claims

1. A care robot (20) comprising:
a holding section (23), provided on a base (21), that is configured to assist a care receiver (M1) in standing up and sitting down by supporting a body part of the care receiver (M1);
a specific information acquisition device (52, 53) that is configured to acquire specific information for identifying the care receiver (M1);
a recognizing section (26) that is configured to recognize the care receiver (M1) from the specific information acquired by the specific information acquisition device (52, 53);
an acquisition section (26) that is configured to acquire information including medication information and/or vital signs information related to the care receiver (M1) recognized by the recognizing section (26) from a host computer (51); and
a control section (26) that is configured to perform drive control of the holding section (23) based on the medication information and/or vital signs information related to the care receiver (M1) acquired by the acquisition section (26) from the host computer (51).

2. The care robot (20) according to claim 1, wherein the specific information acquisition device (52, 53) comprises:
a terminal device (41) that is configured to be worn by the care receiver (M1), has an identification symbol linked to the care receiver (M1), and is configured to send operation instructions for the care robot (20) of claim 1 and an identification symbol of the care receiver (M1) as transmission data, and
a receiver (31) that receives the transmission data from the terminal device (41);
and the recognizing section (26) is configured to recognize the care receiver (M1) from the identification symbol of the care receiver (M1) which is among the transmission data received by the receiver (31).

3. The care robot (20) according to claim 1, wherein the specific information acquisition device (52, 53) comprises:
a terminal device (41) that is configured to be worn by a caregiver (M2) who assists the care receiver (M1), has an identification symbol linked to the caregiver (M2), and is configured to send operation instructions for the care robot (20) of claim 1, an identification symbol of the caregiver (M2), and an identification symbol of the care receiver (M1) as transmission data, and
a receiver (31) that is configured to receive the transmission data from the terminal device (41);
and the recognizing section (26) is configured to recognize the care receiver (M1) from the identification symbol of the care receiver (M1) which is among the transmission data received by the receiver (31).

4. The care robot (20) according to claim 1, wherein the specific information acquisition device (52, 53) is a physical characteristic acquisition device (53) that is configured to acquire a physical characteristic of the care receiver (M1);
and the recognizing section (26) is configured to recognize the care receiver (M1) from the physical characteristic acquired by the physical characteristic acquisition device (53).

5. The care robot (20) according to any one of claims 1 to 4 further comprising:
an operation permission section (26) that, in cases in which the care receiver (M1) and the caregiver (M2) are operators (M1, M2) who operate the care robot according to any of claims 1 to 4, is configured to permit operation when the operator (M1, M2) has operation authority with respect to operation of the care robot (20), and is configured to prohibit operation when the operator (M1, M2) does not have operation authority with respect to operation of the care robot (20).

6. The care robot (20) according to any one of claims 1 to 5, wherein the control section (26) is configured to perform adjustment of speed of the holding section (23) when assisting the care receiver (M1) to stand up or sit down based on information related to the care receiver (M1) acquired by the control section (26).

7. The care robot (20) according to any one of claims 1 to 6 further comprising a traveling drive section (21g, 21h) that is configured to move the base (21).

8. The care robot (20) according to claim 7, wherein the control section (26) is configured to perform adjustment of the moving speed of the base (21) when assisting the movement of the care receiver (M1) by controlling the traveling drive section (21g, 21h) based on information related to the care receiver (M1) acquired by the acquisition section (26).

## Patentansprüche

1. Pflege-Roboter (20), der umfasst:
einen Halte-Teilabschnitt (23), der sich auf einem Untersatz (21) befindet und so ausgeführt ist, dass er einen Pflegebedürftigen (M1) beim Aufstehen und Hinsetzen unterstützt, indem er einen Körperteil des Pflegebedürftigen (M1) stützt;
eine Vorrichtung (52, 53) zur Erfassung spezifischer Informationen, die so ausgeführt ist, dass sie spezifische Informationen zum Identifizieren des Pflegebedürftigen (M1) erfasst;
einen Erkennungs-Teilabschnitt (26), der so ausgeführt ist, dass er den Pflegebedürftigen (M1) anhand der durch die Vorrichtung (52, 53) zur Erfassung spezifischer Informationen erfassten spezifischen Informationen erkennt;
einen Erfassungs-Teilabschnitt (26), der so ausgeführt ist, dass er Informationen, die Medikations-Informationen und/oder Vitalparameter-Informationen bezüglich des durch den Erkennungs-Teilabschnitt (26) erkannten Pflegebedürftigen (M1) einschließen, von einem Host-Computer (51) erfasst; sowie
einen Steuerungs-Teilabschnitt (26), der so ausgeführt ist, dass er Antriebssteuerung des Halte-Teilabschnitts (23) auf Basis der durch den Erfassungs-Teilabschnitt (26) von dem Host-Computer (51) erfassten Medikations-Informationen und/oder Vitalparameter-Informationen bezüglich des Pflegebedürftigen (M1) durchführt.

2. Pflege-Roboter (20) nach Anspruch 1, wobei die Vorrichtung (52, 53) zur Erfassung spezifischer Informationen umfasst:
ein Endgerät (41), das so ausgeführt ist, dass es von dem Pflegebedürftigen (M1) getragen wird, ein mit dem Pflegebedürftigen (M1) verknüpftes Identifikationssymbol aufweist und so ausgeführt ist, dass es Bedienungsanweisungen für den Pflege-Roboter (20) nach Anspruch 1 sowie ein Identifikationssymbol des Pflegebedürftigen (M1) als Übertragungs-Daten sendet, und
eine Empfangsvorrichtung (31), die die Übertragungs-Daten von dem Endgerät (41) empfängt;
und der Erkennungs-Teilabschnitt (26) so ausgeführt ist, dass er den Pflegebedürftigen (M1) anhand des Identifikationssymbols des Pflegebedürftigen (M1) erkennt, das sich unter den durch die Empfangsvorrichtung (31) empfangenen Übertragungs-Daten befindet.

3. Pflege-Roboter (20) nach Anspruch 1, wobei die Vorrichtung (52, 53) zur Erfassung spezifischer Informationen umfasst:
ein Endgerät (41), das so ausgeführt ist, dass es von einer Pflegekraft (M2) getragen wird, die den Pflegebedürftigen (M1) unterstützt, ein mit der Pflegekraft (M2) verknüpftes Identifikationssymbol aufweist und so ausgeführt ist, dass es Bedienungsanweisungen für den Pflege-Roboter (20) nach Anspruch 1, ein Identifikationssymbol der Pflegekraft (M2) sowie ein Identifikationssymbol des Pflegebedürftigen (M1) als Übertragungs-Daten sendet, und eine Empfangsvorrichtung (31), die so ausgeführt ist, dass sie die Übertragungs-Daten von dem Endgerät (41) empfängt;
und der Erkennungs-Teilabschnitt (26) so ausgeführt ist, dass er den Pflegebedürftigen (M1) anhand des Identifikationssymbols des Pflegebedürftigen (M1) erkennt, das sich unter den durch die Empfangsvorrichtung (31) empfangenen Übertragungs-Daten befindet.

4. Pflege-Roboter (20) nach Anspruch 1, wobei die Vorrichtung (52, 53) zur Erfassung spezifischer Informationen eine Vorrichtung (53) zur Erfassung eines physischen Merkmals ist, die so ausgeführt ist, dass sie ein physisches Merkmal des Pflegebedürftigen (M1) erfasst; und der Erkennungs-Teilabschnitt (26) so ausgeführt ist, dass er den Pflegebedürftigen (M1) anhand des durch die Vorrichtung (53) zur Erfassung eines physischen Merkmals erfassten physischen Merkmals erkennt.

5. Pflege-Roboter (20) nach einem der Ansprüche 1 bis 4, der des Weiteren umfasst:
einen Teilabschnitt (26) zum Gestatten von Bedienung, der so ausgeführt ist, dass er, wenn der Pflegebedürftige (M1) und die Pflegekraft (M2) Bedienungspersonen (M1, M2) sind, die den Pflege-Roboter nach einem der Ansprüche 1 bis 4 bedienen, Bedienung gestattet, wenn die Bedienungsperson (M1, M2) über Bedienungsberechtigung in Bezug auf Bedienung des Pflege-Roboters (20) verfügt, und so ausgeführt ist, dass er Bedienung nicht gestattet, wenn die Bedienungsperson (M1, M2) nicht über Bedienungsberechtigung in Bezug auf Bedienung des Pflege-Roboters (20) verfügt.

6. Pflege-Roboter (20) nach einem der Ansprüche 1 bis 5, wobei der Steuerungs-Teilabschnitt (26) so ausgeführt ist, dass er Anpassung von Geschwindigkeit des Halte-Teilabschnitts (23) beim Unterstützen des Pflegebedürftigen (M1) beim Aufstehen oder Hinsetzen auf Basis durch den Steuerungs-Teilabschnitt (26) erfasster Informationen bezüglich des Pflegebedürftigen (M1) durchführt.

7. Pflege-Roboter (20) nach einem der Ansprüche 1 bis 6, der des Weiteren einen Fahrantriebs-Teilabschnitt (21g, 21h) umfasst, der so ausgeführt ist, dass er den Untersatz (21) bewegt.

8. Pflege-Roboter (20) nach Anspruch 7, wobei der Steuerungs-Teilabschnitt (26) so ausgeführt ist, dass er Anpassung der Bewegungsgeschwindigkeit der Basis (21) beim Unterstützen der Bewegung des Pflege-Empfängers (M1) durchführt, indem er den Fahrantriebs-Teilabschnitt (21g, 21h) auf Basis durch den Erfassungs-Teilabschnitt (26) erfasster Informationen bezüglich des Pflege-Empfängers (M1) steuert.

## Revendications

1. Robot de soin (20) comprenant :
une section de maintien (23), disposée sur une base (21), qui est configurée pour fournir une aide à un receveur de soin (M1) pour se lever et s'asseoir en supportant une partie du corps du receveur de soin (M1) ;
un dispositif d'acquisition d'informations spécifiques (52, 53) qui est configuré pour acquérir des informations spécifiques pour identifier le receveur de soin (M1) ;
une section de reconnaissance (26) qui est configurée pour reconnaître le receveur de soin (M1) à partir des informations spécifiques acquises par le dispositif d'acquisition d'informations spécifiques (52, 53) ;
une section d'acquisition (26) qui est configurée pour acquérir des informations incluant des informations de médicament et/ou des informations de signes vitaux liées au receveur de soin (M1) reconnues par la section de reconnaissance (26) provenant d'un ordinateur hôte (51) ; et
une section de commande (26) qui est configurée pour exécuter la commande d'entraînement de la section de maintien (23) sur la base des informations de médicament et/ou les informations de signes vitaux liées au receveur de soin (M1) acquises par la section d'acquisition (26) provenant de l'ordinateur hôte (51).

2. Robot de soin (20) selon la revendication 1, le dispositif d'acquisition d'informations spécifiques (52, 53) comprenant :
un terminal (41) qui est configuré pour être porté par le receveur de soin (M1), qui a un symbole d'identification lié au receveur de soin (M1), et qui est configuré pour envoyer des instructions de fonctionnement pour le robot de soin (20) selon la revendication 1 et un symbole d'identification du receveur de soin (M1) sous la forme de données de transmission, et
un récepteur (31) qui reçoit les données de transmission à partir du terminal (41) ;
et la section de reconnaissance (26) est configurée pour reconnaître le receveur de soin (M1) à partir du symbole d'identification du receveur de soin (M1) qui fait partie des données de transmission reçues par le récepteur (31).

3. Robot de soin (20) selon la revendication 1, le dispositif d'acquisition d'informations spécifiques (52, 53) comprenant :
un terminal (41) qui est configuré pour être porté par le donneur de soin (M2) qui fournit une aide au receveur de soin (M1), qui a un symbole d'identification lié au donneur de soin (M2), et qui est configuré pour envoyer des instructions de fonctionnement pour le robot de soin (20) de la revendication 1, un symbole d'identification du donneur de soin (M2), et un symbole d'identification du receveur de soin (M1) sous la forme de données de transmission, et
un récepteur (31) qui est configuré pour recevoir les données de transmission provenant du terminal (41) ;
et la section de reconnaissance (26) est configurée pour reconnaître le receveur de soin (M1) à partir du symbole d'identification du receveur de soin (M1) qui fait partie des données de transmission reçues par le récepteur (31).

4. Robot de soin (20) selon la revendication 1, le dispositif d'acquisition d'informations spécifiques (52, 53) étant un dispositif d'acquisition de caractéristique physique (53) qui est configuré pour acquérir une caractéristique physique du receveur de soin (M1) ;
et la section de reconnaissance (26) est configurée pour reconnaître le receveur de soin (M1) à partir de la caractéristique physique acquise par le dispositif d'acquisition de caractéristique physique (53).

5. Robot de soin (20) selon l'une quelconque des revendications 1 à 4 comprenant en outre :
une section de permission de fonctionnement (26) qui, dans les cas où le receveur de soin (M1) et le donneur de soin (M2) sont des opérateurs (M1, M2) qui font fonctionner le robot de soin selon l'une quelconque des revendications 1 à 4, est configurée pour permettre le fonctionnement quand l'opérateur (M1, M2) a la permission de fonctionnement relative au fonctionnement du robot de soin (20), et est configurée pour empêcher le fonctionnement quand l'opérateur (M1, M2) n'a pas la permission de fonctionnement relative au fonctionnement du robot de soin (20).

6. Robot de soin (20) selon l'une quelconque des revendications 1 à 5, la section de commande (26) étant configurée pour effectuer un ajustement de la vitesse de la section de maintien (23) durant l'aide fournie au receveur de soin (M1) pour se lever ou s'asseoir sur la base des informations liées au receveur de soin (M1) acquises par la section de commande (26).

7. Robot de soin (20) selon l'une quelconque des revendications 1 à 6 comprenant en outre une section d'entraînement mobile (21g, 21h) qui est configurée pour déplacer la base (21).

8. Robot de soin (20) selon la revendication 7, la section de commande (26) étant configurée pour effectuer un ajustement de la vitesse de déplacement de la base (21) durant l'aide fournie au mouvement du receveur de soin (M1) en commandant la section d'entraînement mobile (21g, 21h) sur la base des informations liées au receveur de soin (M1) acquises par la section d'acquisition (26).
